# EUROPEAN PATENT APPLICATION

(11) **EP 2 168 954 A1**
(43) Date of publication of application: **31.03.2010**
(21) Application number: 08787650.4
(22) Date of filing: 12.06.2008
(51) Int. Cl.: C07D 303/48, C07K 5/06, A61K 31/336, A61K 38/05, A61P 33/06

(54) **CYSTEINE PROTEASES INHIBITORS**

(30) Priority: 15.06.2007 ES 200701717
(71) Applicant: Universitat Jaume I, 12006 Castellón De La Plana (ES)
(72) Inventor: GONZÁLEZ ADELANTADO, Florenci Vicent, E-12001 Grao De Castelló (ES); RODRÍGUEZ PASTOR, Santiago, E-12002 Castelló De La Plana (ES); IZQUIERDO FERRER, Javier, E-12002 Castelló De La Plana (ES)
(74) Representative: ZBM Patents
(86) International application number: PCT/ES2008/070116
(87) International publication number: WO 2008/152178

(57) **Abstract**

Substantially pure diastereoisomeric compounds of formula **Ia,** alternatively **Ib,** or a pharmaceutically acceptable salt thereof or a pharmaceutically acceptable solvate thereof, including hydrates, wherein PG is a protective group; R₁ is a radical selected from the group consisting of phenylmethyl, 4-hydroxyphenylmethyl, (1*H*-indol-3-yl)methyl and (1*H*-imidazol-4-yl)methyl; R₂ is a radical selected from the group consisting of -H, -CH₃, -CH₂SH, -CH₂OH -CH₂Ph, -CH₂CO₂H, -CH₂CONH₂, -CH(OH)CH₃, -CH(CH₃)CH₂CH₃, -CH(CH₃)₂, -CH₂CH(CH₃)₂, -(CH₂)₂SCH₃, -(CH₂)₂CO₂H, -(CH₂)CONH₂, -(CH₂)₃NHC(NH)NH₂, -(CH₂)₄NH₂, imidazol-4-ylmethyl, 4-hydroxyphenylmethyl, (1*H*-indol-3-yl)methyl, (1*H*-imidazol-4-yl)methyl and -(CH₂)ₙ-Ar; and R₃ is a radical selected from the group consisting of -O(C₁-C₄)alkyl, -O(C₂-C₄)alkenyl, -O(C₂-C₄)alkynyl, -O(C₁-C₄)alkyl-Ar, -OAr, - NR^{a}Ar, -N(R^{a})[(C₁-C₄)alkyl-Ar], and -NR^{a}OAr and -N(R^{a})[O(C₁-C₄)alkyl-Ar]. These compounds are inhibitors of cysteine-proteases cruzain, rhodesain and falcipain, and therefore, useful in the treatment and/or prevention of pathologies such as Chagas disease, African trypanosomiasis or malaria.

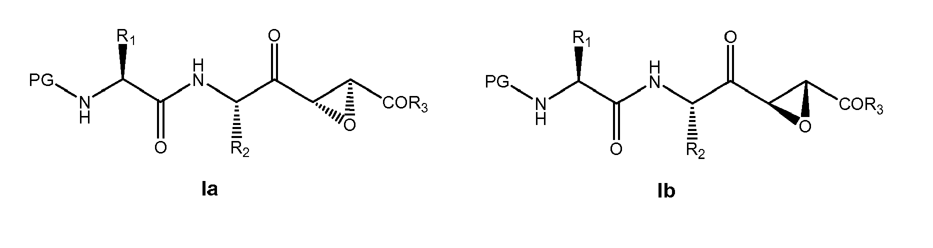

## Description

The present invention relates to new cysteine-protease inhibitors, as well as useful intermediates and processes for their preparation, and pharmaceutical compositions containing them.

### Background of the invention

Trypanosomiasis and malaria are major parasitic diseases in developing countries.

Chagas disease or American trypanosomiasis is a tropical disease caused by the protozoan parasite *Trypanosoma cruzi* which affects between 16 and 18 million people in Latin America causing 50000 deaths every year. Twenty-five per cent of Latin American population (90 million) is reckoned to be at risk of contracting the disease (Weekly Epidemiological Record, World Health Organization, 2000, 2:10-12).

African trypanosomiasis or sleeping sickness affects about half million people in sub-Saharan Africa, and 66 million people in 36 countries are reckoned to be at risk of contracting it. The eastern variant of the disease is caused by *Trypanosoma brucei rhodesiense* and the western variant by *Trypanosoma brucei gambiense,* the infectious agent being in both cases the tsetse fly, which is widely distributed throughout Africa.

Moreover, malaria is caused by protozoa of *Plasmodium species,* mainly *Plasmodium falciparum* and is transmitted to people by anopheles mosquitoes. Malaria affects nearly 40% of world's population where over three thousand million people are at risk, occurring in about one hundred countries from very depressed tropical areas of Africa, Asia and Latin America. Over 90% of cases and 80% of deaths occur in tropical Africa (World Malaria Report 2005).

So far, no vaccine exists for American trypanosomiasis or Chagas disease. The drugs used (benznidazole and nifurtimox) are only useful in the early stages, show low efficacy and undesirable side effects. Furthermore, Melarsoprol is the only available drug for African trypanosomiasis or sleeping sickness. It consists of a toxic arsenic compound, which is administered with its antidote, and exposes 10% of individuals to risk of arsenic encephalopathy, from which almost 1000 people die each year on account of drug toxicity. Recently, eflornithine has been developed for sleeping sickness treatment which, in spite of having proved to be effective and well tolerated, has shown many disadvantages.

Finally, although there are some drugs to prevent malaria such as chloroquine and mefloquine, these show side effects and none of them guarantees the total protection.

Chagas disease, African trypanosomiasis and malaria have a similar infection cycle in which the infectious agent (insect) transmits the pathogen to man. All the pathogens of these diseases possess a common essential enzymatic activity for their life cycle, i.e., cysteine-protease activity: cruzain in *Trypanosoma cruzi,* rhodesain in *T. Brucei* and falcipain in *Plasmodium falciparum* (C.R. Caffrey et al, Mol. Biochem. Parasitol. 2001, vol. 1, pp. 61-73)

Recent investigations directed to the search for drugs for these diseases is based on the inhibition of the above mentioned cysteine-proteases, so that the synthesis of inhibitors to these enzymes could give rise to new drugs for fighting diseases, specially as an alternative to traditional therapy in resistant organisms (J.H. McKerrow et al, Bioorg. Med. Chem. 1999, vol. 7, pp. 639-644).

To this effect, various peptide-based irreversible cysteine-protease inhibitors have been developed, for example, halomethyl ketones, diazomethanes, epoxysuccinyl derivatives, and vinyl sulfone derivatives (H.Otto et al, Chem. Rev. 1997, vol. 97, pp.133-171). A disadvantage of chloromethyl ketones is their high reactivity (they react with serine- proteases and other molecules containing SH groups) and the consequent lack of selectivity thereby resulting in *in vivo* toxicity. In order to increase the selectivity and reduce the reactivity of these compounds, other less reactive molecules have been developed, for example monofluoromethyl ketones (D.Rasnick, Anal. Biochem. 1985, vol. 149, pp. 461-465) and epoxy derivatives (W.R. Roush et al, Bioorg. Med. Chem. Lett. 1998, pp. 2809-2812). However, these compounds are not effective *in vivo* due to their low bioavailability.

Thus, despite the research efforts spent in the past, treatment of such parasitic diseases is far from being satisfactory. Therefore, development of new compounds for the treatment of these disease is still of great interest.

### Description of the invention

The inventors have found new inhibitors of cysteine-protease cruzain, rhodesain and falcipain, which are additionally selective against cathepsin B.

Thus, an aspect of the present invention relates to a substantially pure diastereoisomeric compound of formula **Ia**, alternatively **Ib,** or a pharmaceutically acceptable salt thereof or a pharmaceutically acceptable solvate thereof, including hydrates, 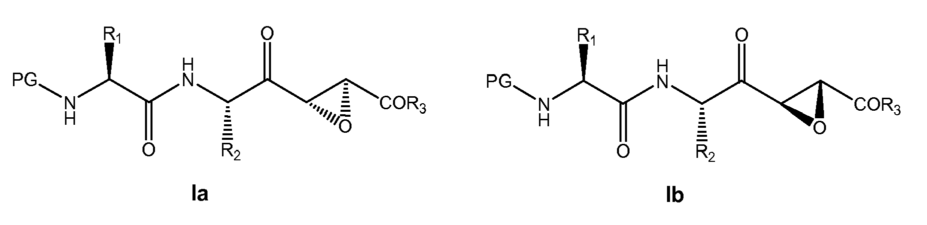 wherein PG is a protective group;
R₁ is a radical selected from the group consisting of phenylmethyl, 4-hydroxyphenylmethyl, (1*H*-indol-3-yl)methyl and (1*H*-imidazol-4-yl)methyl;
R₂ is a radical selected from the group consisting of -H, -CH₃, -CH₂SH, -CH₂OH, -CH₂Ph, -CH₂CO₂H, -CH₂CONH₂, -CH(OH)CH₃, -CH(CH₃)CH₂CH₃, -CH(CH₃)₂, -CH₂CH(CH₃)₂, -(CH₂)₂SCH₃, -(CH₂)₂CO₂H, -(CH₂)CONH₂, -(CH₂)₃NHC(NH)NH₂, -(CH₂)₄NH₂, imidazol-4-ylmethyl, 4-hydroxyphenylmethyl, (1*H*-indol-3-yl)methyl, (1*H*-imidazol-4-yl)methyl and -(CH₂)ₙ-Ar; and
R₃ is a radical selected from the group consisting of -O(C₁-C₄)alkyl, -O(C₂-C₄)alkenyl, -O(C₂-C₄)alkynyl, -O(C₁-C₄)alkyl-Ar, -OAr, -NR^{a}Ar, -N(R^{a})[(C₁-C₄)alkyl-Ar], -NR^{a}OAr and -N(R^{a})[O(C₁-C₄)alkyl-Ar];
wherein n represents a value selected between 2 and 3;
Ar is a carbon or nitrogen radical of a known 5-6 membered carbocyclic aromatic monocyclic ring or an 8-10 membered bicyclic ring optionally containing from 1 to 3 heteroatoms selected from N, S and O, and can be optionally substituted with one or more radicals independently selected from the group consisting of -OH, -CHO, -SH, -NO₂, -CN, -F, -Cl, -Br, -(C₁-C₄)alkyl optionally substituted with one or more radicals independently selected from the group consisting of -F, -Cl, -Br and -OH; -O(C₁-C₄)alkyl optionally substituted with one or more radicals independently selected from the group consisting of -F, -Cl, -Br and -OH; -CO(C₁-C₄)alkyl, -OCO(C₁-C₄)alkyl, -S(C₁-C₄)alkyl, -SO(C₁-C₄)alkyl, -SO₂(C₁-C₄)alkyl, -SO₂O(C₁-C₄)alkyl, -OSO₂(C₁-C₄)alkyl, -NR^{a}R^{b}, -CONR^{a}R^{b}; and
R^{a} and R^{b} independently represent a -H or -(C₁-C₄)alkyl radical.

Some compounds of the invention of formula **Ia,** alternatively **Ib,** in addition to the aforesaid chiral centers, may possess other chiral centers, which can give rise to a variety of stereoisomers. It is an object of the present invention to provide each of the individual stereoisomers as well as their mixtures.

Moreover, some compounds of the present invention may show cis/trans isomery. It is an object of the present invention to provide each of the geometric isomers as well as their mixtures.

The compounds of the invention of formula **Ia,** alternatively **Ib,** are inhibitors of a cysteine-protease selected from the group consisting of cruzain, rhodesain and falcipain.

Therefore, another aspect of the present invention relates to a pharmaceutical composition comprising a therapeutically effective amount of a substantially pure diastereoisomeric compound of formula **Ia,** alternatively **Ib,** or a pharmaceutically acceptable salt thereof or a pharmaceutically acceptable solvate thereof, including a hydrate, together with appropriate amounts of pharmaceutically acceptable excipients.

Another aspect of the present invention relates to the use of the compounds of the invention of formula **Ia,** alternatively **Ib,** for the preparation of a medicament for the treatment and/or prevention of diseases mediated by the inhibition of a cysteine-protease selected from the group consisting of cruzain, rhodesain and falcipain.

Another aspect of the present invention relates to the use of the compounds of the invention of formula **Ia,** alternatively **Ib,** for the preparation of a medicament for the treatment and/or prevention of Chagas disease or African trypanosomiasis. Another aspect of the present invention relates to the use of the compounds of the invention of formula **Ia,** alternatively **Ib,** for the preparation of a medicament for the treatment and/or prevention of malaria.

The invention also relates to a method for the treatment and/or prevention of mammals, including humans, suffering or liable to suffer a disease selected from the group consisting of Chagas disease, African trypanosomiasis and malaria. This method comprises administering to said patients a therapeutically effective amount of a compound of the invention of formula la, alternatively **Ib,** together with pharmaceutically acceptable excipients or carriers.

Another aspect of the present invention relates to a process for the preparation of the compounds of the invention of formula **Ia,** alternatively **Ib,** which comprises oxidation of a compound of formula **IIa,** alternatively **IIb,** with a suitable oxidizing agent: 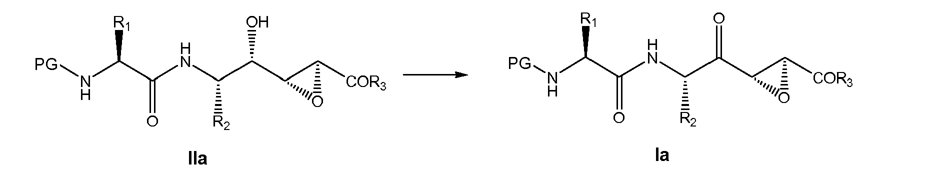 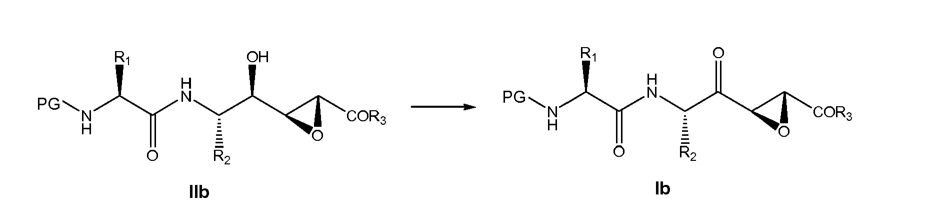 wherein PG, R₁, R₂ and R₃ have the meaning described above.

Another aspect of the present invention relates to a diastereoisomeric intermediate compound of formula **IIa,** alternatively **IIb,** or a pharmaceutically acceptable salt thereof, or a pharmaceutically acceptable solvate thereof, including hydrates, 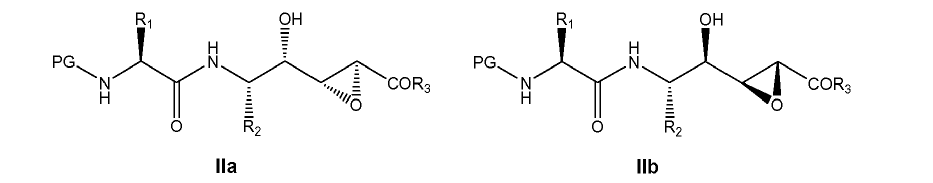 wherein PG, R₁, R₂ and R₃ have the meaning described above.

Another aspect of the present invention relates to a process for the preparation of a compound of the invention of formula **IIa,** alternatively **IIb,** which comprises epoxidation of a compound of formula **IIIa**, alternatively **IIIb,** with a suitable epoxidizing agent: 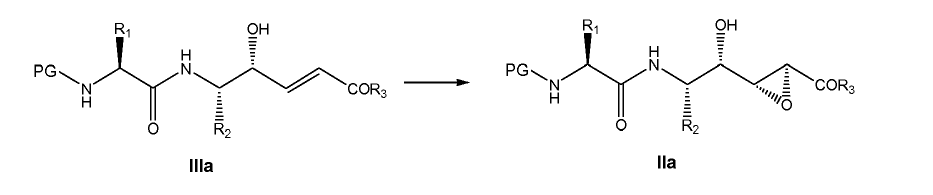 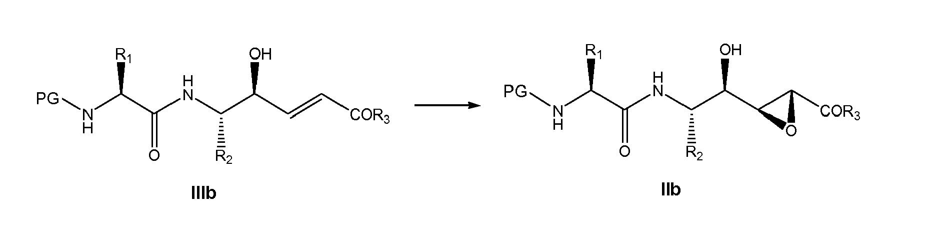 wherein PG, R₁, R₂ and R₃ have the meaning described above.

In the above definitions, the term (C₁-C₄)alkyl means a radical of a linear or branched saturated hydrocarbon chain having 1 to 4 carbon atoms. Examples include, among others, methyl, ethyl, propyl, isopropyl, butyl, isobutyl, *sec-*butyl and *tert*-butyl groups.

The term (C₂-C₄)alkenyl means a radical of a linear or branched unsaturated hydrocarbon chain having 1 to 4 carbon atoms and at least one double bond. Examples include, among others, ethenyl, propenyl, isopropenyl, 1-butenyl, 2-butenyl, 3-butenyl and 1,3-butadienyl groups.

The term (C₂-C₄)alkynyl means a radical of a linear or branched unsaturated hydrocarbon chain having 1 to 4 carbon atoms and at least one triple bond. Examples include, among others, ethinyl, propinyl, isopropinyl, 1-butinyl, 2-butinyl and 3-butinyl groups.

The expression "optionally substituted with one or more substituents" refers to the possibility of a group to be substituted with one or more substituents, preferably with 1, 2, 3 or 4 substituents, whenever said group has 1, 2, 3 or 4 positions susceptible to be substituted.

Ar is a carbon or nitrogen radical of a known 5-6 membered carbocyclic aromatic monocyclic ring or an 8-10 membered bicyclic ring optionally containing from 1 to 3 heteroatoms selected from N, S and O and can be optionally substituted as indicated above. Examples of Ar radicals include, without limitation, thiophenyl, furanyl, pyrrolyl, pyrazolyl, imidazolyl, thiazolyl, isothiazolyl, oxazolyl, isoxazolyl, triazolyl, thiadiazolyl, oxadiazolyl, phenyl, pyridinyl, pyrazinyl, pyridazinyl, pyrimidinyl, triazinyl, thienofuranyl, thienopyrrolyl, pyrrolopyrazolyl, benzothiophenyl, benzofuranyl, indolyl, isoindolyl, benzimidazolyl, benzothiazolyl, imidazopyridinyl, pyrazolopyridinyl, isoquinolinyl, quinolinyl, quinolizinyl, naphthyridinyl, quinazolinyl, quinoxalinyl and naphthyl.

In the context of this invention, the term "substantially pure diastereoisomeric compound" means a compound with sufficient diastereoisomeric excess for its preparation on industrial scale, which depends on each specific case as the person skilled in the art will decide about. In most cases, a diastereoisomeric excess higher than 95% is sufficient.

Although the present invention includes all the compounds mentioned above, a particular embodiment of the invention relates to a substantially pure diastereoisomeric compound of formula **Ia,** or a pharmaceutically acceptable salt thereof, or a pharmaceutically acceptable solvate thereof, including a hydrate.

In another particular embodiment, in a substantially pure diastereoisomeric compound of formula **Ia,** alternatively **Ib,** R₁ represents phenylmethyl; R₂ is a -(CH₂)ₙ-Ar radical; R₃ is a radical selected from the group consisting of -O(C₁-C₄)alkyl, -O(C₁-C₄)alkyl-Ar, -OAr, n represents 2; and Ar is a carbon radical of a known 5-6 membered carbocyclic aromatic monocyclic ring, which optionally contains from 1 to 3 heteroatoms selected from N, S and O.

In another particular embodiment, in a compound of the invention of formula **Ia,** alternatively **Ib,** PG is a radical selected from the group consisting of benzyloxycarbonyl, morpholinocarbonyl and N-methylcarbonyl; R₁ is a phenylmethyl radical, R₂ is a 2-phenylethyl radical and R₃ is a-O(C₁-C₄)alkyl radical.

In another particular embodiment, in a compound of the invention of formula **Ia,** alternatively **Ib,** PG is a benzyloxycarbonyl radical; R₁ is a phenylmethyl radical, R₂ is a 2-phenylethyl radical and R₃ is an ethoxyl radical.

As mentioned above, the invention also relates to the intermediates of formula **IIa,** alternatively **IIb,** their pharmaceutically acceptable salts and their pharmaceutically acceptable solvates, including hydrates.

Thus, another particular embodiment relates to a substantially pure diastereoisomeric intermediate of formula **IIa,** or a pharmaceutical acceptable salt thereof, or a pharmaceutically acceptable solvate thereof, including a hydrate.

In another particular embodiment, in a compound of the invention of formula **IIa,** alternatively **IIb,** R₁ represents phenylmethyl; R₂ is a -(CH₂)ₙ-Ar radical; R₃ is a radical selected from the group consisting of -O(C₁-C₄)alkyl, -O(C₁-C₄) alkyl-Ar, -OAr, n represents 2; and Ar is a carbon radical of a known 5-6 membered carbocyclic aromatic monocyclic ring, which optionally contains from 1 to 3 heteroatoms selected from N, S and O.

In another particular embodiment, in a compound of the invention of formula **IIa,** alternatively **IIb,** PG is a radical selected from the group consisting of benzyloxycarbonyl, morpholinocarbonyl and N-methylcarbonyl; R₁ is a phenylmethyl radical, R₂ is a 2-phenylethyl radical and R₃ is a -O(C₁-C₄)alkyl radical.

In another particular embodiment, in a compound of the invention of formula **IIa,** alternatively **IIb,** PG is a benzyloxycarbonyl radical; R₁ is a phenylmethyl radical, R₂ is a 2-phenylethyl radical and R₃ is an ethoxyl radical.

In addition, all possible combinations of the embodiments described above also form part of the invention.

The compounds of the present invention may contain one or more basic nitrogen atoms and, therefore, may form salts with acids, both organic and inorganic acids, which also form part of the present invention. Examples of said salts include: salts with inorganic acids such as hydrochloric acid, hydrobromic acid, hydroiodic acid, nitric acid, perchloric acid, sulfuric acid or phosphoric acid; and salts with organic salts, such as methanesulfonic acid, trifluoromethanesulfonic acid, ethanesulfonic acid, benzenesulfonic acid, p-toluenesulfonic acid, fumaric acid, oxalic acid, acetic acid or maleic acid, among others.

The compounds of the present invention may contain one or more acidic protons and, therefore, might form salts with bases, which also form part of the present invention. Examples of said salts include: salts with inorganic cations such as sodium, potassium, calcium, magnesium, lithium, aluminium, zinc, etc.; salts formed with pharmaceutically acceptable amines such as ammonia, alkylamines, hydroxyalkylamines, lysine, arginine, N-methylglucamine, procaine and the like. There is no limitation in the nature of these salts provided that they are pharmaceutically acceptable when used for therapeutic purposes. The salts may be prepared by treating the compound of formula **Ia, Ib, IIa** or **IIb** with a sufficient amount of the desired acid or base to give the salt in a conventional form. The salts of the compounds of formula la, **Ib, IIa** or **IIb** may be converted in turn into other salts of the compounds of formula **Ia, Ib, IIa** or **IIb** respectively, by ion exchange using an ion-exchange resin.

The present invention also includes pharmaceutical compositions. The chosen composition will depend upon the nature of administered compound and its route of administration. Different routes of drug administration may be used, for example oral or topical administration.

Solid compositions for oral administration include tablets, granules and capsules. The method for preparation may be based on a simple mixture, dry granulation or wet granulation of the active ingredient with excipients. These excipients may be, for example, diluents, binders, disintegrants, and lubricants. Tablets may be coated with appropriate excipients in order to delay their disaggregation or absorption in the gastrointestinal tract or improve their organoleptic properties or their stability. The active ingredient may also be incorporated by coating on inert pellets by using natural or synthetic film-forming polymers.

In case soft gelatin capsules are used, the active ingredient is mixed with water or with oily medium. Oral suspensions of powders or granules may be prepared by the addition of water, mixing the active ingredient with dispersing or humectant agents; suspending and preserving agents. Other excipients, for example sweeteners, flavoring agents and coloring agents may also be added.

As liquid forms for oral administration, emulsions, solutions, suspensions, syrups and elixirs containing commonly used inert diluents may be included. These compositions may also contain coadjuvants such as humectants, suspending agents, sweeteners, flavoring agents, preserving agents, and pH regulators.

The compounds may also be formulated for its topical administration. Topical formulations include creams, lotions, gels, ointments, powders, solutions and patches in which the compound has been dispersed or dissolved in suitable excipients.

The substantially pure diastereoisomeric compounds of formula **Ia,** alternatively **Ib,** may be prepared by the processes hereinafter described. It will be evident to a person skilled in the art that the precise method used for the preparation of a certain compound can vary depending on its chemical structure.
If not otherwise specified, the meanings of variables PG, R₁, R₂ and R₃ are those indicated above.

Moreover, it may be necessary or convenient to protect the reactive or labile groups by conventional protective groups in some of the aforesaid processes. Both the nature of said protective groups and the processes for their introduction or removal are well known and form part of prior art (Greene T.W. and Wuts P.G.M, "Protective Groups in Organic Synthesis", John Wiley & Sons, 3rd edition, 1999). Some examples of protective groups for an amino function that can be used are 9-fluorenylmethoxy-carbonyl (Fmoc), *tert-*butoxycarbonyl (Boc), benzyl (Bn), benzyloxycarbonyl (Cbz), trifluoroacetyl (TFA), morpholinocarbonyl and N-methylcarbonyl groups. The hydroxyl groups can be protected for example with trimethylsilyl (TMS), *tert-*butyldimethylsilyl (TBS) or tetrahydropyranyl (THP) groups. The reaction for deprotection of these groups is carried out under the usual conditions of organic synthesis, such as those described in the aforesaid reference.

A substantially pure diastereoisomeric compound of formula **Ia** can be obtained by oxidation of a compound of formula **IIa.** This reaction is carried out in the presence of an oxidizing agent, for example, Dess-Martin periodinane, in a suitable solvent, for example, dichloromethane, at an appropriate temperature, preferably room temperature.

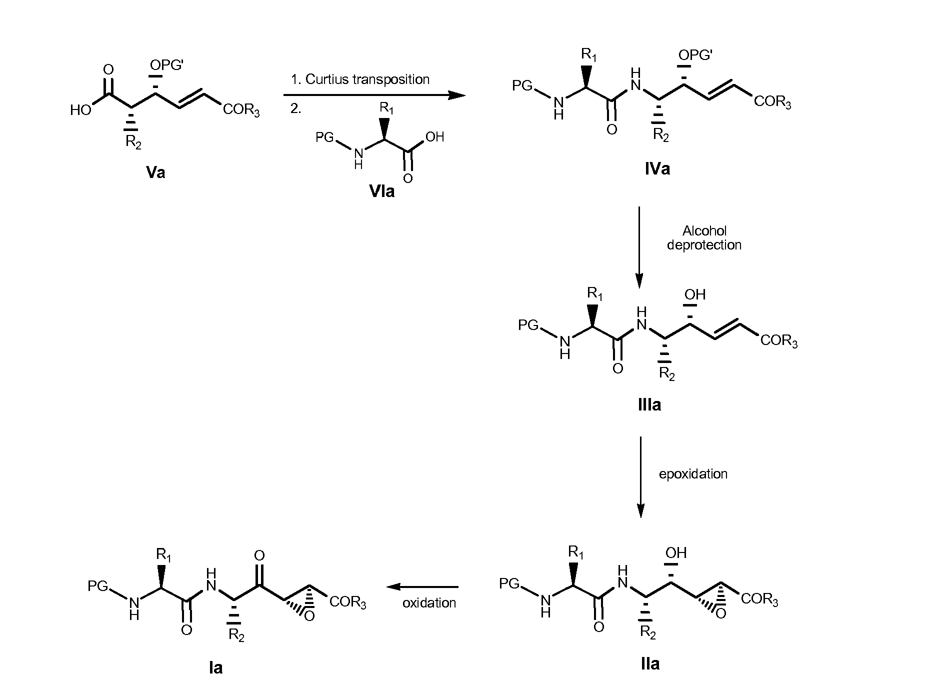

A compound of formula **IIa** can be obtained by epoxidation reaction of an allylic alcohol of formula **IIIa.** This reaction is carried out in the presence of an epoxidizing agent, such as lithium *tert*-butylhydroperoxide, in a suitable solvent, for example, tetrahydrofuran, at an appropriate temperature, preferably room temperature. Under these conditions, the *syn*-epoxy alcohol is mostly obtained in relation to the hydroxyl group (S. Rodriguez et al, Tetrahedron 2006, pp.11112-11123).

A compound of formula **IIIa** can be prepared from a carboxylic acid of formula **Va**, wherein PG' is a hydroxyl-protective group, for example *tert-*butyldimethylsilyl, by a sequence of various steps. In a first step, the carboxylic acid compound of formula **Va** is converted into an amino group by Curtius transposition. This reaction can be carried out using diphenylphosphoryl azide, in the presence of a base, such as for example, triethylamine, in a suitable solvent, for example, *N*,*N*-dimethylformamide, at an appropriate temperature, preferably room temperature, to form an isocyanate intermediate. Alternatively, the isocyanate intermediate can be formed by reacting the carboxylic acid of formula **Va** with methyl chloroformate and subsequently sodium azide.

Aqueous treatment of the obtained isocyanate intermediate at suitable temperature, preferably at 100 °C, gives rise to a primary amine. In a second step, the amine obtained in the first step is converted into an amide of formula **IVa** by reaction with an amino acid of formula **VIa** with the amino group conveniently protected if necessary, for example, protected with the benzyloxycarbonyl group. This reaction can be carried out in the presence of a suitable condensing agent, for example, *N*-(3-dimethylaminopropyl)-*N*'-ethylcarbodiimide or dicyclohexylcarbodiimide optionally in the presence of 1-hydroxybenzotriazol, or in the presence of suitable base, such as 4-dimethylaminopyridine or pyridine, in a suitable solvent, such as dichloromethane or dimethylformamide.

Alternatively, the isocyanate obtained in the first step is converted into an amide of formula **IVa** by reaction with an amino acid of formula **VIa** with the amino group conveniently protected if necessary, for example, protected with the benzyloxycarbonyl group. This reaction can be carried out in the presence of suitable base, such as 4-dimethylaminopyridine, in a suitable solvent, such as dichloromethane.

A compound of formula **Va** can be obtained from a compound of formula **XIII** following the synthetic pathway shown in the following scheme:

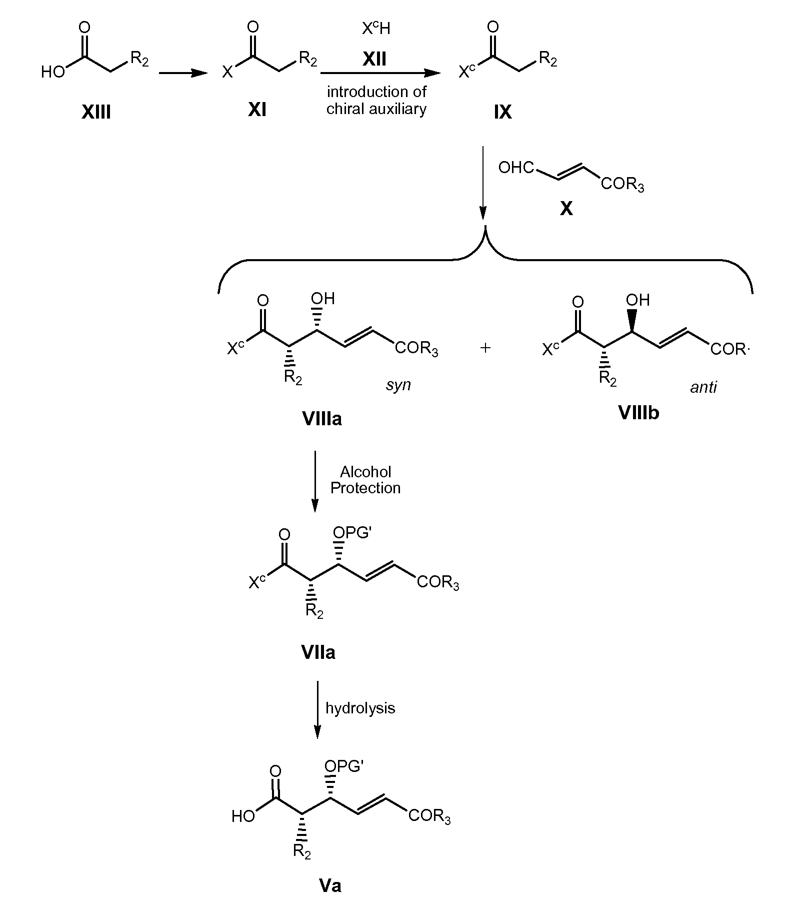

The carboxylic acid of formula **XIII** is converted into an acylated derivative of formula **XI** wherein X represents halogen, preferably chloro, by reaction with a halogenating agent, for example, thionyl chloride, in a suitable solvent, for example, benzene, at an appropriate temperature, preferably by heating.

Subsequently, the compound of formula **XI** is converted into a compound of formula **IX** by reaction with a compound of formula **XII,** wherein X^{c} represents a chiral auxiliary, for example, (S)-4-benzyl-2-thioxothiazolidin-3-yl. This reaction is carried out in the presence of a base, for example, triethylamine or 2,6-lutidine and optionally in the presence of *tert*-butyldimethylsilyl triflate (TBSOTf), in a suitable solvent, for example, dichloromethane or without solvent and at an appropriate temperature, for example, room temperature.

The asymmetric aldol reaction between the ketone of formula **IX** and an aldehyde of formula X gives rise to *syn* and *anti aldols* of formula **VIIIa** and **VIIIb** respectively, which have a defined absolute stereochemisty and are separable by chromatography. This reaction can be carried out, for example, as described in D.A. Evans et al, Org. Lett. 2002, vol. 4, pp. 1127-1130; D.A. Evans et al, J. Am. Chem. Soc. 2002, vol. 124, pp. 392-393 and D.A. Evans et al, J. Am. Chem. Soc. 1981, vol. 103, pp. 2127-2129. When X^{c} is an (S)-4-benzyl-2-thioxothiazolidin-3-yl radical and the reaction is carried out as described in D.A. Evans et al, Org. Lett. 2002, 4, 1127, the compound of formula **VIIIb** is mostly obtained.

After separating the compounds of formula **VIIIa** and **VIIIb,** the hydroxyl group of the compound of formula **VIIIa** can be protected to give a compound of formula **VIIa,** wherein PG' represents a protective group, for example, *tert-*butyldimethylsilyl. This reaction is carried out under conditions which are well known to the person skilled in the art, for example, those described in Greene T.W. and Wuts P.G.M, "Protective Groups in Organic Synthesis", John Wiley & Sons, 3rd edition, 1999.

Hydrolysis of a compound of formula **VIIa** gives rise to a compound of formula Va. This reaction can be carried out in the presence of hydrogen peroxide and a base, for example, lithium hydroxide, in a suitable solvent, for example, a tetrahydrofuran-water mixture and at an appropriate temperature, preferably room temperature.

Compounds **XIII, XII, X** and **VIa** are commercially available or can be easily obtained by conventional methods.

A substantially pure diastereoisomeric compound of formula **Ib** can be obtained in an analogous way to that used for a compound of formula **Ia** from the corresponding aldol of formula **VIIIb:**

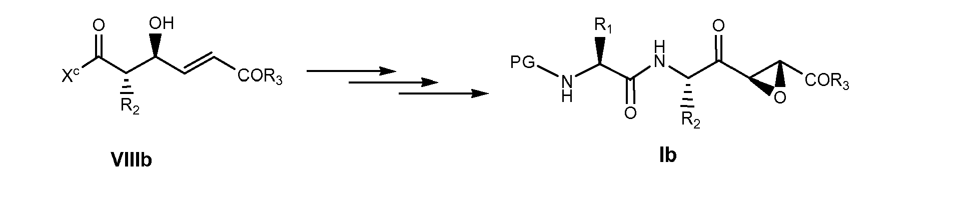

Moreover, a compound of formula **Ia,** alternatively **Ib,** can be converted into another compound of formula **"Ia,** alternatively **Ib.** For example, a compound of formula **Ia,** alternatively **Ib,** wherein R₃ represents -O(C₁-C₄)alkyl, -O(C₁-C₄)alkyl-Ar or -OAr, can be converted into a compound of formula la, alternatively **Ib,** wherein R₃ represents -NHAr or -NH-OAr, by reactions which are well known to the person skilled in the art. Likewise, the interconversion reactions of an R³ radical into another R³ radical can be also carried out through any of the intermediate of the compounds of the invention of formula **Ia,** alternatively **Ib.**

Throughout the description and claims the word "comprise" and variations of the word are not intended to exclude other technical features, additives, components, or steps.

Additional objects, advantages and features of the invention will become apparent to those skilled in the art partly upon examination of the description and partly by the practice of the invention. The following examples are provided by way of illustration, and they are not intended to be limiting of the present invention.

### EXAMPLES

All the solvents used in the reactions were distilled at the time of being used by means of suitable drying agents. ¹H-NMR and ¹³C-NMR spectra were recorded in CDCl₃ solution (¹H, 7.24 ppm; ¹³C 77.0 ppm) at 30 °C on a 300 MHz Mercury Varian or 500 MHz Innova Varian RMN spectrometer at the "Serveis Centrals d'Instrumentació Científica de la Universitat Jaume I". Mass spectra were recorded on a QTOF I mass spectrometer (quadrupole-hexapole-TOF) with orthogonal Z-spray-electrospray interface (Micromass, Manchester, UK). IR spectra were recorded on oily films on NaCl tablets in a Perkin-Elmer 2000 FT-IR spectrometer. For column chromatography, EM Science Silica Gel 60 was used, whereas TLC was performed on E. Merck silica gel coated aluminum foils (Kieselgel 60, F₂₅₄, 0.25 mm). Except when indicated otherwise, all the reactions were carried out under nitrogen atmosphere with magnetic stirring.

The following abbreviations are used in the below examples:
AcOEt: Ethyl acetate
AMC: 7-Amino-4-methyl-coumarin
Arg: L-Arginine
4-DMAP: 4-*N*,*N*-dimethylaminopyridine
DMSO: dimethyl sulfoxide
DTT: dithiothreitol
Et₃N: triethylamine
Et₂O: diethyl ether
Hex: hexane
HPhe: L-Homophenylalanine
Phe: L-Phenylalanine
TBHP: *tert*-butyl hydroperoxide
TBSOTf: *tert*-butyldimethylsilyl triflate
THF: tetrahydrofuran
TMSCI: trimethylsilyl chloride
TX-100: triton-X (benzyltrimethylammonium hydroxide)
Z: benzyloxycarbonyl

Intermediate **XI.1** (R₂ = 2-phenylethyl; X = Cl): **4-phenylbutanoyl chloride.**

A solution of 4-phenylbutyric acid (18.76g, 114.23mmol) in benzene (147mL) was treated with thionyl chloride (25.27mL, 348mmol) and the resulting mixture was refluxed for 2h, then the solvent was removed under vacuum and the resulting brown oil was subjected to the following step without further purification.

Intermediate **IX.1** (R₂ = 2-phenylethyl, X^{c}: (S)-4-benzyl-2-thioxothiazolidin-3-yl): **1-((*S*)-4-benzyl-2-thioxothiazolidin-3-yl)-4-phenylbutan-1-one.**

A solution cooled in an ice bath of (*S*)-4-benzylthiazolidin-2-thione (18.37g, 87.87mmol) in CH₂Cl₂ (266mL) was treated with Et₃N (2 eq) and then with 4-phenylbutanoyl chloride (Intermediate **XI.1,** 114.23mmol). The resulting mixture was stirred at room temperature for 12 days and then quenched with brine and extracted with CH₂Cl₂, dried with Na₂SO₄, filtered and concentrated. Purification by silica gel chromatography using Hex:AcOEt mixtures of increasing polarity (95:5, 9:1, 8:2, 7:3 and 6:4) afforded 24g (70%) of a yellow oil.

¹³C-NMR (500MHz, CDCl₃) δ 201.06, 173.73, 141.53, 136.56, 129.44, 128.88, 128.53, 128.36, 127.19, 125.96, 68.53, 37.93, 36.79, 35.11, 31.90, 26.43.

Intermediates **VIIIa.1** (*syn*) **and VIIIb.1** (*anti*) (R₂ = 2-phenylethyl, R₃ = ethoxyl, X^{c}: (*S*)-4-benzyl-2-thioxothiazolidin-3-yl): **Ethyl (2*E*,4*R*,5*S*)-5-((4*S*)-4-benzyl-2-thioxothiazolidin-3-carbonyl)-7-phenyl-4-hydroxyhept-2-enoate** and **Ethyl (2*E*,4*S*,5*S*)-5-((4*S*)-4-benzyl-2-thioxotiazolidin-3-carbonyl)-7-phenyl-4-hydroxyhept-2-enoate.**

MgBr·EtO₂ (440.47mg, 1.71mol) was introduced into a two-necked flask with magnetic stirring bar inside the drybox. Then a solution of 1-((*S*)-4-benzyl-2-thioxothiazolidin-3-yl)-4-phenylbutan-1-one (Intermediate **IX.1,** 4.79g, 13.12mmol) in AcOEt (33mL), ethyl (E)-3-formylacrylate (Intermediate **X.1** (R₃ = ethoxyl), 5.04g, 39.35mmol), Et₃N (4.02mL, 28.86mmol) and TMSCI (2.83mL, 22.30mmol) were sequentially added and the resulting mixture was stirred at room temperature for 12 days. Afterwards, the crude was filtered through silica gel using Et₂O as eluent and concentrated under vacuum. The resulting crude was dissolved in THF (200mL) and treated with aqueous 1 M HCl (46mL). The resulting mixture was stirred at room temperature for 1.5h, then quenched with solid NaHCO₃, filtered and concentrated. Purification by silica gel chromatography using Hex:AcOEt mixtures of increasing polarity (8:2, 1:1, 1:2, 1:4 and 1:7) afforded the desired compounds as yellow oils (weight of the two compounds jointly: 3.80 g (60%)).

Intermediate **VIIIa.1** (*sin*): ¹H-NMR (500MHz, CDCl₃) δ 7.20-7.35 (m, 10H), 6.94 (1H, dd, J= 15.5 and 3.5Hz), 6.14 (1H, dd, J= 15.5 and 1.0Hz), 5.14 (1H, m), 4.73 (1H, m), 4.46 (1H, m), 4.08 (3H, m), 3.31 (1H, dd, J= 12.0 and 7.5Hz), 3.08 (1H, dd, J= 13.0 and 3.5Hz), 2.94 (1H, dd, J= 13.5 and 10.5Hz), 2.80 (1H, m), 2.60 (m, 2H), 2.45 (1H, m), 2.15 (m, 1H), 1.85 (m, 1H), 1.22 (3H, t, J= 7.0Hz).

Intermediate **VIIIb.1** (*anti*): ¹H-NMR (500MHz, CDCl₃) δ 7.10-7.25 (m, 10H), 6.84 (1H, dd, J= 15.5 and 3.5Hz), 6.03 (1H, dd, J= 15.5 and 1.0Hz), 5.24 (1H, m), 4.83 (1H, m), 4.55 (1H, m), 4.17 (3H, m), 3.24 (1H, dd, J= 11.0 and 6.5Hz), 3.03 (1H, dd, J= 13.5 and 4.0Hz), 2.91 (1H, dd, J= 13.0 and 10.0Hz), 2.76 (2H, m), 2.57 (m, 1H), 2.12 (m, 1H), 2.03 (m, 1H), 1.19 (3H, t, J= 7.0Hz).

Intermediates **VIIa.1** (*syn*) and **VIIb.1** (*anti*) (R₂ = 2-phenylethyl, R₃ = ethoxyl, X^{c}: (*S*)-4-benzyl-2-thioxothiazolidin-3-yl, PG': *tert*-butyldimethylsilyl): Ethyl **(2*E*,4*R*,5*S*)-5-((4*S*)-4-benzyl-2-thioxothiazolidin-3-carbonyl)-4-*tert-*butyldimethylsilyloxy-7-phenylhept-2-enoate** and **Ethyl (2*E*,4*S*,5*S*)-5-((4*S*)-4-benzyl-2-thioxothiazolidin-3-carbonyl)-4-*tert-*butyldimethylsilyloxy-7-phenylhept-2-enoate.**

A solution of aldol (intermediate **VIIIb.1,** 3.63g, 8.05mmol) cooled at -70°C in CH₂Cl₂ (80mL) was treated with 2,6-lutidine (3.75mL, 32.19) and TBSOTf (5.53mL, 24.15mmol). The resulting mixture was stirred at the above temperature for 5.5h, and then was quenched with a saturated aqueous solution of NaHCO₃, extracted with CH₂Cl₂, dried (Na₂SO₄), filtered and concentrated. Purification by silica gel chromatography using Hex:AcOEt mixtures of increasing polarity (7:3 and 1:1) afforded 4.47g (93%) of a yellow oil corresponding to intermediate **VIIb.1.**

Following a similar process, as described above, but using intermediate **VIIIa.1** as starting material, compound **VIIa.1** was obtained.

Intermediate **VIIa.1** (*syn*): IR δ 3063, 3027, 2954, 2930, 2857, 1718, 1659, 1604, 1496, 1471, 1455, 1365, 1341, 1262, 1193, 1161, 1135, 1083, 1083, 1039, 983, 939, 883, 838, 779, 747, 701, 667 cm⁻¹.

Intermediate **VIIb.1** (*anti*): IR δ 3063, 3027, 2930, 2857, 1716, 1659, 1604, 1496, 1455, 1367, 1341, 1159, 1038, 984, 837, 779, 746, 701, 667 cm⁻¹.

Intermediates **Va.1** (*syn*) **and Vb.I** (*anti*) (R₂ = 2-phenylethyl, R₃ = ethoxyl, PG': *tert*-butyldimethylsilyl): **(*E*,2*S*,3*R*)-5-(Ethoxycarbonyl)-3-*tert-*butyldimethylsilyloxy-2-phenethylpent-4-enoic acid** and **(*E*,2*S*,3*S*)-5-(Ethoxycarbonyl)-3-tert-butyldimethylsilyloxy-2-phenethylpent-4-enoic** acid.

To a solution of protected aldol (intermediate **VIIb.1,** 2.02g, 4.98mmol) in THF-H₂O (3:1) (50mL) cooled in an ice bath, H₂O₂ (30%) (3.08mL, 29.90mmol) and then a solution of LiOH (238mg, 9.96mmol) in water (2mL) were added dropwise. The resulting mixture was stirred at room temperature for 2 h. Afterwards, the reaction was quenched with Na₂SO₃(1.6M aqueous solution) (8mL) and stirred for 20min, then concentrated under vacuum and the resulting aqueous solution was extracted with CH₂Cl₂, dried (Na₂SO₄), filtered and concentrated. Purification by silica gel chromatography using Hex:AcOEt mixtures of increasing polarity (7:3, 6:4 and 1:1) afforded 1.86 g (83%) of a yellowish oil corresponding to intermediate **Vb.1**.

Following a similar process, as described above, but using intermediate **VIIa.1** as starting material, compound **Va.1** was obtained.

Intermediate **Va.1** (*syn*): ---

Intermediate **Vb.1** (*anti*): ¹HRMS *mlz* calcd. for C₂₂H₃₄O₅SiNa [M+Na⁺]: 429.2073, found: 429.2099.

Intermediates **IVa.1** (*syn*) and **IVb.1** (*anti*) (PG = benzyloxycarbonyl, R₁ = benzyl, R₂ = 2-phenylethyl, R₃ = ethoxyl, PG': *tert*-butyldimethylsilyl): **Ethyl (2*E*,4*R*,5*S*)-5-((2*S*)-2-benzyloxycarbonylamino-3-phenylpropionylamino)-4-*tert*-butyldimethylsilyloxy-7-phenylhept-2-enoate and Ethyl (2*E*,4*S*,5*S*)-5-((2*S*)-2-benzyloxycarbonylamino-3-phenylpropionylamino)-4-*tert-*butyldimethylsilyloxy-7-phenylhept-2-enoate.**

A solution of carboxylic acid (intermediate **Vb.1,** 969mg, 2.38mmol) in acetone-H₂O (12mL) cooled in an ice bath was treated with Et₃N (389µL, 2.79mmol) and then with methyl chloroformate (247µL, 3.19mmol).The resulting mixture was stirred at this temperature for 1.5h. Then a solution of NaN₃ (309mg, 4.76mmol) was added in water (1.5mL) and the mixture was stirred -cooled in an ice bath- for 4h, then poured into a decantation funnel and extracted with Et₂O, dried (Na₂SO₄), filtered and concentrated. The resulting yellow oil was dissolved in toluene (12mL) and refluxed for 35min, then concentrated under vacuum and the crude was subjected to the following step without further purification.
Afterwards, a solution of the above crude and (*S*)-benzyloxycarbonyl-phenylalanine (911 mg, 3.05mmol) in CH₂Cl₂ (35mL) cooled in an ice bath was treated with 4-DMAP (71 mg, 0.58mmol). The resulting mixture was stirred at this temperature for 1.5h and then at room temperature for 6h. The mixture was then concentrated under vacuum. Purification by silica gel chromatography using Hex:AcOEt mixtures of increasing polarity (6:4) afforded 752mg (48%) of an oil corresponding to intermediate **IVb.1.**

Following a similar process, as described above, but using intermediate **Va.1** as starting material, compound **IVa.1** was obtained.

Intermediate **IVa.1** (*syn*): ¹³C-NMR (500MHz, CDCl₃) δ 173.91, 165.43, 147.09, 141.25, 136.03, 129.00, 128.45, 127.92, 126.77, 125.50, 122.10, 73.98, 68.60, 60.13, 49.00, 36.43, 32.65, 31.17, 30.34, 25.23, 13.73, -4.63, - 5.44.

Intermediate **IVb.1** (*anti*): IR δ 3321, 3027, 2929, 2858, 1719, 1659, 1535, 1497, 1455, 1366, 1260, 1174, 1130, 1031, 837, 778, 746, 698 cm⁻¹.

Intermediates **IIIa.1** (*syn*) and **IIIb.1** (*anti*) (PG = benzyloxycarbonyl, R₁ = benzyl, R₂ = 2-phenylethyl, R₃ = ethoxyl): **Ethyl (2*E*,4*R*,5*S*)-5-((2*S*)-2-benzyloxycarbonylamino-3-phenyl-propionylamino)-7-phenyl-4-hydroxyhept-2-enoate** and **Ethyl (2*E*,4*S*,5*S*)-5-((2*S*)-2-benzyloxycarbonylamino-3-phenylpropionylamino)-7-phenyl-4-hydroxy-hept-2-enoate.**

To a solution of protected compound (intermediate **IVb.1,** 725mg, 1.10mmol) in THF (11 mL) cooled in an ice bath, tetra-*n*-butylammonium fluoride (1 M in THF) (5.5mL, 5.5mmol) was added. The resulting mixture was stirred at room temperature for 7.5 h. Afterwards, the solvent was removed under vacuum and the resulting crude was purified by silica gel chromatography using Hex:AcOEt mixtures of increasing polarity (1:1) affording 467mg (78%) of a white solid corresponding to intermediate **IIIb.1.**

Following a similar process, as described above, but using intermediate **IVa.1** as starting material, compound **IIIa.1** was obtained.

Intermediate **IIIa.1** (*syn*): ¹³C-NMR (500MHz, CDCl₃) δ 171.98, 166.44, 156.39, 145.97, 141.32, 136.69, 136.27, 129.00, 128.45, 127.92, 126.77, 125.60, 73.36, 67.50, 60.76, 54.07, 38.73, 32.64, 30.67, 14.44.

Intermediate **IIIb.1** *(anti):* IR δ 3438, 3309, 3029, 2927, 1690, 1642, 1532, 1495, 1455, 1385, 1369, 1302, 1258, 1219, 1190, 1135, 1041, 971, 911, 873, 750, 700, 673 cm⁻¹.

Intermediates **IIa.1** (*syn-syn*) and **IIb.1** (*anti-syn*): (PG = benzyloxycarbonyl, R₁ = benzyl, R₂ = 2-phenylethyl, R₃ = ethoxyl): **Ethyl (2*S*,3*R*)-3-[(1*S*,2*S*)-2-((2*S*)-2-benzyloxycarbonylamino-3-phenylpropionylamino)-4-phenyl-1-hydroxybutyl]oxirane-2-carboxylate** and **Ethyl (2*R*,3*S*)-3-[(1*R*,2*S*)-2-((2*S*)-2-benzyloxycarbonylamino-3-phenylpropionylamino)-4-phenyl-1-hydroxybutyl]oxirane-2-carboxylate.**

To a solution of TBHP (3.3M in toluene, prepared according to Hill, J.G.; Rossiter, B.E.; Sharpless, B.; J. Org. Chem. 1983, 48, 3607-3608; 616µL, 2.34 mmol) in THF (5 mL) cooled at -78°C was added ethyl lithium (0.5M in benzene/cyclohexane (9/1)) (3.43mL, 1.72 mmol). The resulting mixture was stirred at -78°C for 15 min and then a solution of the unsaturated ester (intermediate **IIIb.1,** 425mg, 0.78 mmol) in THF (3 mL) was added dropwise and the resulting mixture was stirred at room temperature for 3 days. Then Na₂SO₃ (120mg) was added all at once and stirred for 15 min. Later, the mixture was diluted with a saturated aqueous solution of NH₄Cl and extracted with Et₂O (3 x 30 mL), the organic phases were washed with brine, dried and concentrated. The resulting crude oil was purified by silica gel chromatography using Hex:AcOEt mixtures of increasing polarity (7:3), (6:4), (1:1), (1:2) and AcOEt) affording 175mg of a white solid corresponding to intermediate **IIb.1.**

Following a similar process, as described above, but using intermediate **IIIa.1** as starting material, compound **IIa.1** was obtained.

Intermediate **IIa.1** (*syn-syn*): HRMS *mlz* calcd. for C₂₂H₃₄O₅SiNa [M+Na⁺]: 429.2073, found: 429.2099.

Intermediate **IIb.1** (*anti-syn*): ¹³C-NMR (500MHz, CDCl₃) δ 171.73, 168.60, 155.81, 141.11, 136.46, 136.06, 129.31, 129.27, 128.87, 128.83, 128.57, 128.52, 128.36, 128.27, 127.14, 126.14, 70.71, 67.26, 61.80, 58.49, 56.63, 53.05, 51.29, 50.05, 38.06, 33.07, 32.40, 32.30, 32.24, 29.72, 14.14.

Compounds **Ia.1** and **Ib.1** (PG = benzyloxycarbonyl, R₁ = benzyl, R₂ = 2-phenylethyl, R₃ = ethoxyl): **Ethyl (2*S*,3*S*)-3-[(2*S*)-2-((2*S*)-2-benzyloxycarbonylamino-3-phenylpropionylamino)-4-phenylbutyryl] oxirane-2-carboxylate** and **Ethyl (2*R*,3*R*)-3-[(2*S*)-2-((2*S*)-2-benzyloxycarbonylamino-3-phenylpropionylamino)-4-phenylbutyryl] oxirane-2-carboxylate.**

A solution of epoxy alcohol (intermediate **IIb,** 50mg, 0.09mmol) in CH₂Cl₂ (3mL) cooled in an ice bath was treated with pyridine (36µL, 0.45mmol) and Dess-Martin periodinane (169mg, 0.45mmol). The resulting mixture was stirred at room temperature for 4 h, then quenched with a saturated aqueous solution of NaHCO₃/Na₂S₂O₃, diluted with Et₂O, and extracted with Et₂O (3x20mL). The organic phases were washed (brine), dried (Na₂SO₄) and concentrated. The crude was purified by silica gel chromatography using Hex:AcOEt mixtures of increasing polarity (8:2, 7:3, 6:4, 1:1 and AcOEt) to afford 34mg (68 %) of a white solid corresponding to intermediate **IIb.1.**

Following a similar process, as described above, but using intermediate **IIIa.1** as starting material, compound **IIa.1** was obtained.

Compound **Ia.1:** HRMS *m*/*z* calcd. for C₃₂H₃₄O₅N₂O₇Na [M+Na⁺]: 581.2264, found: 581.2251.

Compound **Ib.1:** HRMS m/z calcd. for C₃₂H₃₄O₅N₂O₇Na [M+Na⁺]: 581.2264, found: 581.2258.

### Enzymatic Inhibition Studies and IC₅₀ Determination

Inhibition of cysteine-proteases cruzain, rhodesain, cathepsin B (Tb CatB) and falcipain (FP2) was assessed by quantification of the resulting fluorescence emission of the proteolytic breakdown of synthetic compound Z-Phe-Arg-AMC (Bachem). Inhibitors la or **Ib** were used at concentrations ranging form 10 nM to 10.000 nM (from 500 nM to 10000nM for Tb CatB); thus, serial dilutions in DMSO were prepared with the minimal concentration of inhibitor **Ia** or **Ib** used for calculations and at least 10 times higher than that of the enzyme. These experiments were performed by distributing inhibitor la or **Ib** in 96 wells, fluorescence was recorded on a Flex Station fluorimeter (Molecular Devices), 355 nm excitation and 460 nm emission at 25°C, distributing 100 µl of substrate using a multichannel pipette at a similar volume of the inhibitor enzyme preincubated at 25°C for 5 minutes. In each series of experiments, the enzyme with DMSO and the enzyme in the presence of the known irreversible inhibitor K11777 (N methyl piperazine-Phe-HPhe-(CH=CHSO₂ Ph)), Arris Pharmaceuticals Inc., South San Francisco, CA) were used as controls.

Enzyme inhibition data were similarly determined: recombinant cruzain at 4 nM with 5µM of Z-Phe-Arg-AMC (Km=1µM) in buffer A (100 mM of sodium acetate, pH 5.5 with 5 mM of DTT and 0.001 % Triton-X 100 (Sigma)); recombinant rhodesain at 4 nM of enzyme and 5 µM Z-Phe-Arg-AMC (Kₘ = 1 µM) in buffer A; recombinant Tb CatB at 40 nM of enzyme and 5 µM of Z-Phe-Arg-AMC (Kₘ = 60 µM) in buffer A and recombinant FP2 at 4 nM with 5µM of Z-Phe-Arg-AMC (Km=1 µM) in buffer A. IC₅₀ values were determined by direct reading of the semi-log plot of reaction speed versus inhibitor concentration.

Inhibition tests on *Trypanosoma brucei brucei* were performed by ATP-bioluminescence (Z.B. Mackey et al, Chem. Biol. Drug Des. 2006, 67, pp. 355-363).

Table 1 shows percent inhibition values corresponding to *Trypanosoma brucei brucei* (Tbb), cruzain, rhodesain cathepsin B.

**Table 1**

| **Example** | **Tbb** | **% inhibition (DMSO)** | | |
|---|---|---|---|---|
| | | **Cruzain** | **Rhodesain** | **TbCat B** |
| **Ia.1** | 42% | 93% | 98% | 51% |
| **Ib.1** | -2% | 55% | 80% | 47% |

Tabla 2 shows IC₅₀ values of enzymes falcipain, cruzain, rhodesain and TbCat B.

**Table 2**

| **Example** | **IC₅₀ (nM)** | | | |
|---|---|---|---|---|
| | **Falcipain** | **Cruzain** | **Rhodesain** | **TbCat B** |
| I**a.1** | 71 | 20 | 3.5 | >>1000 |
| **Ib.1** | 44 | 50 | 30 | 400 |

Compounds **Ia.1** and **Ib.1** are very active against rhodesain and cruzain but not against cathepsin B, which indicates their selectivity.

Effectiveness of the compounds of the invention can also be estimated by cellular tests or *in vivo* animal models. Animal models in trypanosomiasis, Chagas disease and malaria are known in prior art.

## Claims

1. A substantially pure diastereoisomeric compound of formula la, alternatively **Ib,** or a pharmaceutically acceptable salt thereof or a pharmaceutically acceptable solvate thereof, including a hydrate, 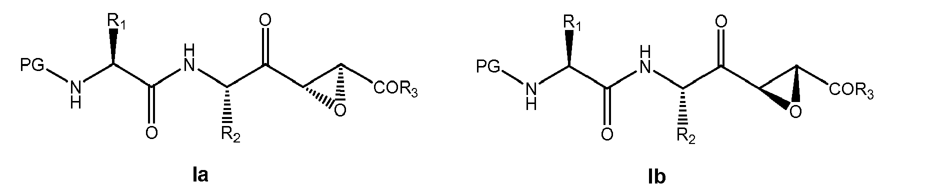 wherein PG is a protective group;
R₁ is a radical selected from the group consisting of phenylmethyl, 4-hydroxyphenylmethyl, (1*H*-indol-3-yl)methyl and (1*H*-imidazol-4-yl)methyl;
R₂ is a radical selected from the group consisting of -H, -CH₃, -CH₂SH, -CH₂OH, -CH₂Ph, -CH₂CO₂H, -CH₂CONH₂, -CH(OH)CH₃, -CH(CH₃)CH₂CH₃, -CH(CH₃)₂, -CH₂CH(CH₃)₂, -(CH₂)₂SCH₃, -(CH₂)₂CO₂H, -(CH₂)CONH₂, -(CH₂)₃NHC(NH)NH₂, -(CH₂)₄NH₂, imidazol-4-ylmethyl, 4-hydroxyphenylmethyl, (1*H*-indol-3-il)methyl, (1*H*-imidazol-4-yl)methyl and -(CH₂)ₙ-Ar; and
R₃ is a radical selected from the group consisting of -O(C₁-C₄)alkyl, -O(C₂-C₄)alkenyl, -O(C₂-C₄)alkynyl, -O(C₁-C₄)alkyl-Ar, -OAr, -NR^{a}Ar, -N(R^{a})[(C₁-C₄)alkyl-Ar], -NR^{a}OAr and -N(R^{a})[O(C₁-C₄)alkyl-Ar];
wherein n represents a value selected between 2 and 3;
Ar is a carbon or nitrogen radical of a known 5-6 membered carbocyclic aromatic monocyclic ring or an 8-10 membered bicyclic ring optionally containing from 1 to 3 heteroatoms selected from N, S and O, and can be optionally substituted with one or more radicals independently selected from the group consisting of -OH, -CHO, -SH, -NO₂, -CN, -F, -Cl, -Br, -(C₁-C₄)alkyl optionally substituted with one or more radicals independently selected from the group consisting of -F, -Cl, -Br and -OH; -O(C₁-C₄)alkyl optionally substituted with one or more radicals independently selected from the group consisting of -F, -Cl, -Br and -OH; -CO(C₁-C₄)alkyl, -OCO(C₁-C₄)alkyl, -S(C₁-C₄)alkyl, -SO(C₁-C₄)alkyl, -SO₂(C₁-C₄)alkyl, -SO₂O(C₁-C₄)alkyl, -OSO₂(C₁-C₄)alkyl, -NR^{a}R^{b}, -CONR^{a}R^{b}; and
R^{a} and R^{b} independently represent a -H or -(C₁-C₄)alkyl radical.

2. A compound according to claim 1 of formula **Ia,** or a pharmaceutically acceptable salt thereof, or a pharmaceutically acceptable solvate thereof, including a hydrate.

3. A compound according to any one of the claims 1-2 wherein PG is a radical selected from the group consisting of benzyloxycarbonyl, morpholinocarbonyl and N-methylcarbonyl; R₁ is a phenylmethyl radical, R₂ is a 2-phenylethyl radical and R₃ is a -O(C₁-C₄)alkyl radical.

4. A compound according to claim 3, wherein PG is a benzyloxycarbonyl radical; R₁ is a phenylmethyl radical, R₂ is a 2-phenylethyl radical and R₃ is an ethoxyl radical.

5. A pharmaceutical composition comprising a therapeutically effective amount of a compound as defined in any one of the claims 1 to 4, or a pharmaceutically acceptable salt thereof, or a pharmaceutically acceptable solvate thereof, including a hydrate together with appropriate amounts of pharmaceutically acceptable excipients.

6. Use of a compound as defined in any one of the claims 1 a 4 for the preparation of a medicament for the treatment and/or prevention of diseases mediated by inhibition of a cysteine-protease selected from the group consisting of cruzain, rhodesain and falcipain.

7. Use of a compound as defined in any one of the claims 1 a 4 for the preparation of a medicament for the treatment and/or prevention of a disease selected from the group consisting of Chagas disease, African trypanosomiasis and malaria.

8. A process for preparing a substantially pure diastereoisomeric compound of formula **Ia,** alternatively **Ib,** or a pharmaceutically acceptable salt thereof, or pharmaceutically acceptable solvate thereof, including a hydrate, which comprises oxidation of a compound of formula **IIa,** alternatively **IIb** with a suitable oxidizing agent: 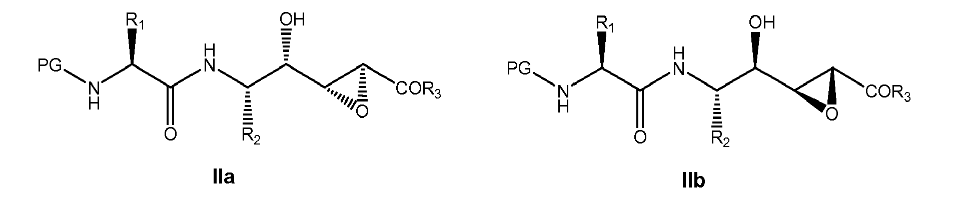 wherein PG, R₁, R₂ and R₃ have the same meaning as in claim 1.

9. A diastereoisomeric compound of formula **IIa,** alternatively **IIb,** or a pharmaceutically acceptable salt thereof or a pharmaceutically acceptable solvate thereof, including a hydrate, 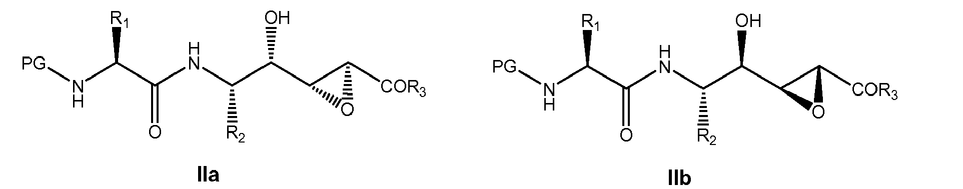 wherein PG, R₁, R₂ and R₃ have the same meaning as in claim 1.

10. A compound according to claim 9 of formula **IIa,** or a pharmaceutically acceptable salt thereof or a pharmaceutically acceptable solvate thereof, including a hydrate.

11. A compound according to any one of the claims 9 to 10 wherein PG is a radical selected from the group consisting of benzyloxycarbonyl, morpholinocarbonyl and N-methylcarbonyl; R₁ is a phenylmethyl radical, R₂ is a 2-phenylethyl radical and R₃ is a -O(C₁-C₄)alkyl radical.

12. A compound according to any one of the claims 9 to 11 wherein PG is a benzyloxycarbonyl radical; R₁ is a phenylmethyl radical, R₂ is a 2-phenylethyl radical and R₃ is an ethoxyl radical.

13. A process for preparing a diastereoisomeric compound of formula **IIa,** alternatively **IIb,** or a pharmaceutically acceptable salt thereof or a pharmaceutically acceptable solvate thereof, including a hydrate, which comprises epoxidation of a compound of formula **IIIa** or **IIIb** respectively with a suitable epoxidizing agent: 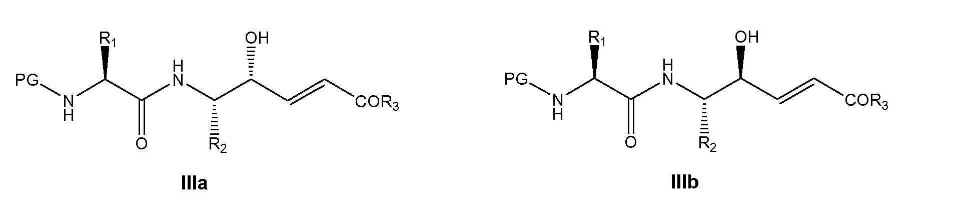 wherein PG, R₁, R₂ and R₃ have the same meaning as in claim 1.
